# FASCICULE DE BREVET EUROPEEN

(11) **EP 3 157 578 B1**
(45) Date de publication et mention de la délivrance du brevet: **17.10.2018**
(21) Numéro de dépôt: 15729493.5
(22) Date de dépôt: 17.06.2015
(51) Int. Cl.: A61L 2/07

(54) **DISPOSITIF DE RINÇAGE ET DE STÉRILISATION D'UN TUYAU DE TIRE-LAIT, ET PROCÉDÉ CORRESPONDANT**
VORRICHTUNG ZUM SPÜLEN UND STERILISIEREN EINES BRUSTPUMPENSCHLAUCHS UND ZUGEHÖRIGES VERFAHREN
DEVICE FOR RINSING AND STERILIZING A BREAST PUMP TUBE, AND CORRESPONDING METHOD

(30) Priorité: 17.06.2014 FR 1455560
(43) Date de publication de la demande: 26.04.2017
(73) Titulaire: Dorel France, 49309 Cholet Cedex (FR)
(72) Inventeur: LEMERCIER, Marie-Agnès, F-14320 Feugeurolles-Bully (FR); BEAUFILS, Yohann, F-14740 Saint Manvieux Norrey (FR); LECLERE, Jean-Marc, F-49450 Villedieu La Blouère (FR); RICARD, Adrien, F-49300 Cholet (FR)
(74) Mandataire: Vidon Brevets & Stratégie
(86) Numéro de dépôt international: PCT/EP2015/063642
(87) Numéro de publication internationale: WO 2015/193397

(56) Documents cités:
- EP-A1- 0 634 177
- EP-A1- 0 679 406
- GB-A- 2 136 911
- US-A- 5 562 882

## Description

### 1. Domaine de l'invention

Le domaine de l'invention est celui de la puériculture.

Plus précisément, l'invention concerne les tire-lait, notamment les tire-lait électriques. Plus précisément encore, l'invention concerne un dispositif de nettoyage d'un tel tire-lait, et en particulier des tubes dans lesquels circule le lait.

### 2. Solutions de l'art antérieur

Il est connu, pour tirer du lait maternel, d'utiliser des dispositifs tire-lait tels que les tire-lait manuels. Ces derniers mettent en oeuvre un levier solidaire d'un réservoir pour recueillir le lait exprimé, le levier étant configuré pour appliquer une dépression entre le réservoir et la poitrine de l'utilisatrice, et ainsi exprimer le lait mammaire.

On connaît également les tire-lait électriques qui comprennent une unité d'aspiration (ou pompe) reliée à une téterelle destinée à être appliquée sur la poitrine, et une unité de collecte du lait exprimé. La plupart du temps, cette unité de collecte est placée à proximité directe de la téterelle, de façon que le lait s'écoule directement dans celle-ci. Cependant, l'utilisatrice est obligée de porter en permanence l'unité de collecte et la pompe associée à proximité de sa poitrine.

Sur certains modèles de tire-lait électriques, il a donc été envisagé de déporter la pompe et/ou l'unité de collecte. Pour ceci, il doit être prévu un tuyau de transfert du lait reliant la téterelle à l'unité de collecte.

De tels appareils étant destinés à collecter du lait maternel pour l'alimentation des nourrissons ou des enfants en bas âge, ils doivent être lavés et stérilisés aisément, notamment afin d'éviter toute transmission de bactéries ou autres germes.

Deux types de nettoyage sont recommandés en fonction de la durée entre deux utilisations du tire-lait. Ainsi, il est recommandé de stériliser les éléments, et notamment le tuyau de transfert du lait, lorsque l'intervalle de temps entre deux utilisations est supérieur à 8 heures. Pour une durée entre deux utilisations inférieure à 8 heures, un rinçage après chaque utilisation peut être suffisant (une stérilisation quotidienne est recommandée toutefois).

Le nettoyage de tels tire-lait est généralement effectué selon l'une des deux techniques suivantes.

Après démontage de tous les éléments du tire-lait, la première technique connue consiste à rincer et laver les éléments, et notamment le tuyau de transfert du lait, à l'eau propre (ajout de savon possible), puis à stériliser les éléments en les trempant dans de l'eau portée à ébullition pendant environ cinq à dix minutes, puis à sécher les éléments à l'aide d'un chiffon propre ou à les laisser sécher à l'air libre.

Un inconvénient de cette première technique est qu'elle met en oeuvre de nombreuses étapes, et qu'elle est donc relativement fastidieuse. En outre, comme indiqué précédemment, il peut être nécessaire de procéder au nettoyage du tire-lait jusqu'à dix fois par jour lors d'une utilisation répétée du dispositif.

Cette technique n'est donc pas satisfaisante.

Un autre inconvénient de cette technique est la consommation en eau importante qu'elle nécessite. En effet, les étapes de rinçage, lavage et stérilisation nécessitent une quantité d'eau relativement importante, voire non négligeable lorsque le nettoyage se répète plusieurs fois dans une même journée. Ceci peut engendrer un surcoût pour les utilisateurs.

La seconde technique connue consiste à utiliser un stérilisateur vapeur adapté aux dispositifs et articles de puériculture. Ces stérilisateurs sont prévus pour stériliser des biberons ou des tétines, mais aussi dans certains cas des éléments d'un tire-lait, ces derniers étant disposés « en vrac » dans le stérilisateur.

Un tel stérilisateur ne permet pas de laver et de rincer tous les éléments d'un tire-lait, et en particulier le tuyau.

Un inconvénient de ces deux techniques est que le lavage et la stérilisation du tuyau de transfert du lait n'est pas optimale, du fait que la section du tuyau est faible et que l'eau ou la vapeur ne circule pas forcément à l'intérieur du tuyau.

Par ailleurs, aucune de ces deux techniques n'est adaptée à la fois au lavage et à la stérilisation des éléments du tire-lait, et notamment du tuyau de transfert du lait.

En d'autres termes, il n'existe actuellement aucun dispositif permettant de combiner un lavage et une stérilisation du tuyau de transfert du lait d'un tire-lait.

EP 0 634 177 A1 concerne un autoclave et un procédé correspondant destinés à la stérilisation d'articles. L'autoclave comprend des moyens de passage d'un fluide de stérilisation à travers l'article. Cependant, cet autoclave n'est pas destiné à un usage domestique au jour le jour, par une mère allaitant son enfant.

### 3. Objectifs de l'invention

L'invention a notamment pour objectif de pallier au moins certains de ces différents inconvénients de l'art antérieur.

Plus précisément, dans au moins un mode de réalisation, un objectif de l'invention est de fournir un dispositif permettant le rinçage et la stérilisation du tuyau de passage du lait d'un tire-lait.

Un autre objectif de l'invention est, selon au moins un mode de réalisation, de fournir un tel dispositif qui soit simple d'utilisation, compact, simple à fabriquer, efficace, robuste, peu coûteux et rapide.

L'invention a aussi pour objectif de fournir, selon au moins un mode de réalisation, un tel dispositif qui ne nécessite qu'une quantité d'eau limitée, tout en assurant un rinçage et une stérilisation optimales.

### 4. Exposé de l'invention

Ces objectifs, ainsi que d'autres qui apparaîtront plus clairement par la suite, sont atteints à l'aide d'un dispositif de rinçage et de stérilisation d'au moins un tuyau de passage du lait d'un tire-lait, notamment d'un tire-lait électrique.

Selon l'invention, ledit dispositif comprend un réservoir et un bouchon permettant de le fermer, ledit réservoir présentant des moyens de raccordement d'une des extrémités du ou d'au moins un desdits tuyaux, placés sur une paroi latérale dudit réservoir, à un niveau intermédiaire entre la paroi de fond et le bord supérieur dudit réservoir, de façon que, lorsque ledit réservoir contient un liquide comprenant de l'eau et que celui-ci est porté à ébullition, la vapeur produite déplace au moins une partie du volume de liquide dans ledit au moins un tuyau permettant son rinçage, puis, lorsque le niveau dudit liquide a atteint lesdits moyens de raccordement, circule dans ledit au moins un tuyau permettant sa stérilisation.

Ainsi, l'invention propose de nettoyer et de stériliser un ou plusieurs tuyaux de passage du lait d'un tire-lait avec un même dispositif.

Pour ce faire, le dispositif de l'invention comprend un réservoir étanche de stockage d'un liquide (par exemple de l'eau) sur lequel on peut raccorder un ou plusieurs tuyau(x) de passage du lait à nettoyer et/ou stériliser (du lait étant présent à l'intérieur de ce(s) tuyau(x) après une utilisation du tire-lait) de façon à ce que le(s) tuyau(x) communique(nt) avec le réservoir.

Le raccordement du ou des tuyaux se fait sur la paroi latérale du réservoir à un niveau intermédiaire entre la paroi de fond (correspondant à un niveau minimum) et le bord supérieur du réservoir, ou une zone voisine du bord supérieur (correspondant à un niveau maximum).

De cette façon, lorsque le liquide est porté à ébullition (en plaçant le dispositif dans un four micro-ondes par exemple), la vapeur formée dans le réservoir étanche déplace une quantité d'eau chaude provenant du réservoir dans le tuyau, ce qui permet le rinçage de ce dernier, puis lorsque le niveau d'eau atteint un seuil prédéterminé (se situant sous le niveau intermédiaire des moyens de raccordement du réservoir avec le tuyau), la vapeur circule alors dans le tuyau, ce qui permet une stérilisation de ce dernier.

L'eau, puis la vapeur, circulent ainsi d'une extrémité du tuyau à l'autre extrémité permettant un rinçage puis une stérilisation optimale de ce dernier en une seule opération.

L'approche de l'invention nécessite uniquement une quantité de liquide prédéterminée et une source de chaleur permettant de porter le liquide à ébullition.

Selon un aspect particulier de l'invention, lesdits moyens de raccordement sont situés à une hauteur comprise entre 40 et 60% de la hauteur totale dudit réservoir.

Cette position peut varier (et dans une variante, être réglable), en fonction des durées et/ou efficacités respectives souhaitées pour la circulation de liquide puis de vapeur dans le ou les tuyaux. Elle prend également en compte le cas échéant la forme et le volume du réservoir.

Selon un aspect particulier de l'invention, ledit dispositif comprend en outre un réceptacle de collecte dudit liquide souillé et de la vapeur ayant circulés dans ledit au moins un tuyau.

Selon un aspect particulier de l'invention, le réceptacle de collecte comprend des moyens de maintien d'une seconde extrémité dudit au moins un tuyau sur ledit réceptacle de collecte.

Le dispositif de l'invention est ainsi configuré pour permettre le passage de l'eau, puis de la vapeur, depuis le réservoir vers ledit réceptacle de collecte, via ledit au moins un tuyau à nettoyer.

Les moyens de raccordement et/ou les moyens de maintien peuvent notamment être adaptés pour permettre le nettoyage et la stérilisation :
- d'un unique tuyau, une première extrémité de ce tuyau étant raccordée au réservoir;
- d'un tuyau ayant une forme générale en Y, les deux branches du Y destinées à être solidarisées chacune à une téterelle étant raccordés au réservoir ou au réceptacle, selon les modes de réalisation ;
- de deux tuyaux distincts, une première extrémité de chacun de ces tuyaux étant raccordée au réservoir ;
- de plus de deux tuyaux, une première extrémité de chacun de ces tuyaux étant raccordée au réservoir, par exemple dans le cas d'un ensemble formant un Y, constitué de trois tronçons de tuyau reliés entre eux de façon amovible par une pièce de liaison.

Selon un aspect particulier de l'invention, lesdits moyens de maintien se présentent sous la forme d'une ouverture située en partie supérieure dudit réceptacle de collecte.

Selon un aspect particulier de l'invention, ledit bouchon porte le ou est solidaire dudit réceptacle de collecte.

Le réservoir comprend ainsi un bouchon qui intègre le réceptacle de collecte ou qui est indépendant de ce dernier.

Selon un aspect particulier de l'invention, ledit réservoir comprend des premiers moyens de solidarisation aptes à coopérer avec des seconds moyens de solidarisation portés par ledit bouchon.

Ces moyens de solidarisation peuvent se présenter sous la forme d'un filetage et d'un taraudage. Ces derniers assurent l'étanchéité du réservoir.

Selon un aspect particulier de l'invention, ledit dispositif comprend une embase portant au moins ledit réservoir.

Selon un aspect particulier de l'invention, ladite embase comprend au moins un élément définissant un espace de rangement dudit au moins un tuyau le long dudit réservoir.

Ladite embase peut présenter au moins une gorge apte à maintenir ledit au moins un tuyau.

Il peut par exemple s'agir d'une gorge hélicoïdale ménagée sur la paroi et autour du réservoir. Une telle gorge peut être mise en oeuvre seule ou en complément d'au moins un élément délimitant l'espace de rangement.

Selon un aspect particulier de l'invention, le réceptacle de collecte comprend un couvercle amovible.

Ceci permet, lorsque le bouchon est ôté, de vider aisément l'eau sale accumulée dans le réceptacle de collecte et ayant servi au rinçage et à la stérilisation du tuyau.

Selon un aspect particulier de l'invention, le couvercle dudit réceptacle de collecte comprend au moins un évent débouchant vers l'extérieur dudit dispositif.

L'invention concerne, par ailleurs, un procédé de rinçage et de stérilisation d'au moins un tuyau de passage de lait d'un tire-lait à l'aide du dispositif de rinçage et de stérilisation tel que décrit précédemment.

Selon l'invention, le procédé comprend une étape de chauffage d'un liquide comprenant de l'eau, contenu dans un réservoir présentant des moyens de raccordement d'une des extrémités du ou d'au moins un desdits tuyaux, placés sur une paroi latérale dudit réservoir, à un niveau intermédiaire entre la paroi de fond et le bord supérieur dudit réservoir, ladite étape de chauffage portant ledit liquide à ébullition de façon à provoquer la production de vapeur, assurant successivement :
- une étape de rinçage, par passage d'au moins une partie du liquide dans ledit au moins un tuyau ; puis
- une étape de stérilisation, par passage de vapeur dans ledit au moins un tuyau.

Selon un aspect particulier de l'invention, ladite étape de stérilisation est suivie d'une et/ou en partie simultanée avec une étape de séchage dudit tuyau, par passage de vapeur dans ledit tuyau.

Selon un aspect particulier de l'invention, ledit procédé comprend en outre :
- une étape de fixation dudit tuyau sur ledit réservoir par le biais des moyens de raccordements ;
- une étape de remplissage dudit réservoir avec un volume de liquide comprenant de l'eau ;
- une étape d'assemblage dudit réceptacle de collecte avec ledit réservoir ;
- une étape de solidarisation dudit tuyau audit réceptacle de collecte par le biais des moyens de maintien.

### 5. Liste des figures

D'autres caractéristiques et avantages de l'invention apparaîtront plus clairement à la suite de la lecture et la description suivante, donnée à titre de simple exemple illustratif et non limitatif, et des dessins annexés ci-dessous, parmi lesquels :
- la figure 1 illustre une vue en perspective du dispositif de rinçage et de stérilisation selon un mode de réalisation de l'invention ;
- la figure 2 est une vue en coupe longitudinale du dispositif de rinçage et de stérilisation de la figure 1 ;
- les figures 3A, 3B et 3C illustrent les différentes étapes d'utilisation du dispositif de rinçage et de stérilisation de l'invention ;
- la figure 4 une vue en perspective du dispositif de rinçage et de stérilisation selon l'invention sans le tuyau et sans le couvercle ;
- les figures 5A et 5B sont des vues éclatées du dispositif de rinçage et de stérilisation selon l'invention, sans et avec le tuyau respectivement ;

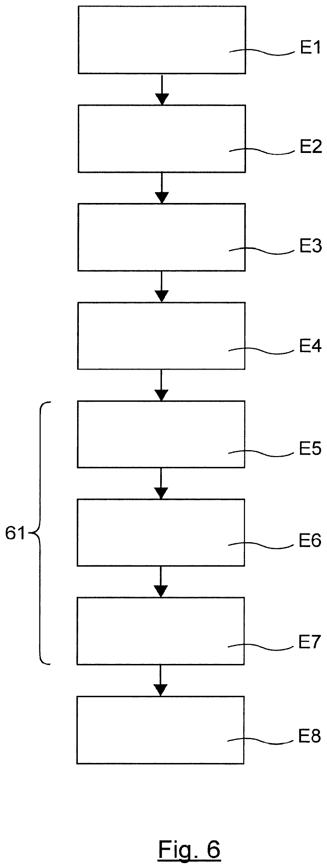

- la figure 6 illustre de façon schématique les étapes d'un exemple de mise en oeuvre du procédé conforme à l'invention.

### 6. Description détaillé d'un mode de réalisation de l'invention

### 6.1. Eléments du dispositif de l'invention

L'invention propose donc un dispositif de rinçage à l'eau et de stérilisation par vapeur d'eau d'au moins un tuyau de passage du lait d'un tire-lait.

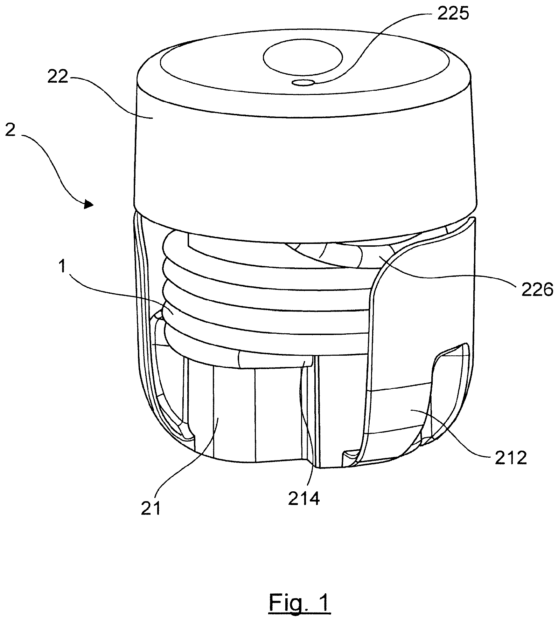

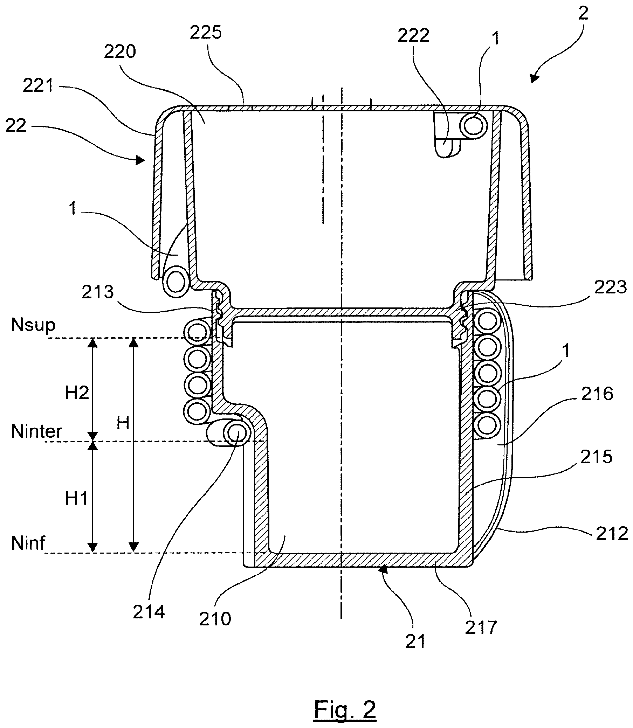

Comme illustré sur le mode de réalisation des figures 1 et 2, le dispositif 2 de rinçage et de stérilisation de l'invention comprend :
- une partie inférieure, ou embase, 21 prévue pour stocker un liquide propre, et plus particulièrement de l'eau propre (du robinet par exemple), cette eau étant destinée à rincer et stériliser un tuyau 1 de passage du lait d'un tire-lait ; et
- une partie supérieure (optionnelle), ou bouchon, 22 qui est montée sur l'embase 21 et agencée pour recueillir le liquide provenant de l'embase 21 et ayant servi au rinçage et à la stérilisation du tuyau 1.

Dans ce mode de réalisation de l'invention, l'embase 21 est de section sensiblement ronde. On comprend bien évidemment que la section ronde de l'embase 21 est un exemple non limitatif et que l'on peut prévoir de multiples variantes.

L'embase 21 du dispositif 2 comprend une partie cylindrique ouverte à une extrémité et fermée à l'autre extrémité par une paroi de fond 217, les deux extrémités étant reliées par une paroi latérale 215, qui est de section sensiblement circulaire dans cet exemple.

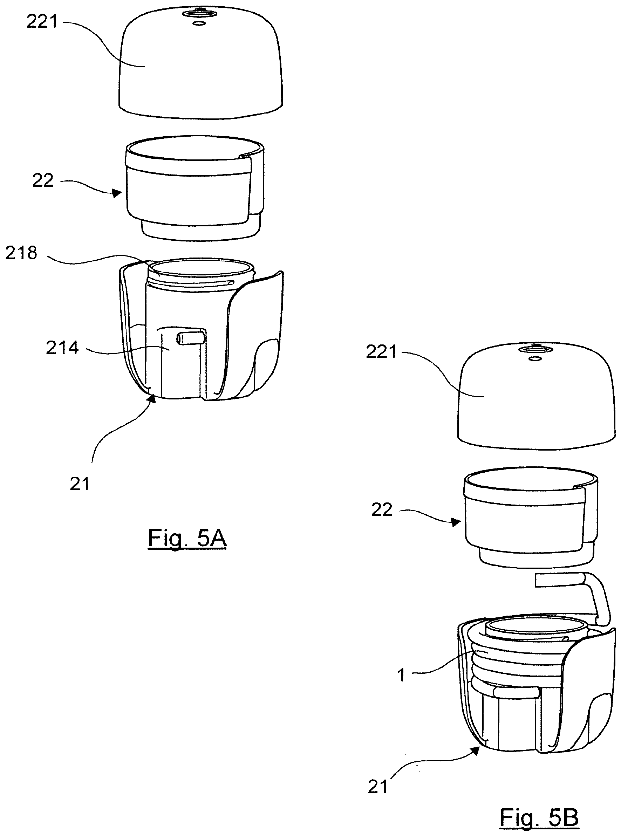

Cette partie cylindrique forme un réservoir 210 apte à recevoir un volume d'eau propre. Le réservoir 210 comprend ainsi une large ouverture à son extrémité supérieure, délimitée par un bord supérieur 218 (figure 5A), de manière à ce que l'utilisateur puisse verser de l'eau propre (ou tout autre liquide adapté) dans le réservoir 210 à chaque utilisation du dispositif 2.

Par ailleurs, l'embase 21 comprend un espace de rangement 216 défini entre la paroi latérale 215 cylindrique et une ou plusieurs lames 212 (par exemple en arc de cercle) s'étendant parallèlement à cette paroi latérale 215, et configuré pour recevoir le tuyau 1 de passage de lait du tire-lait que l'utilisateur souhaite laver/rincer et stériliser.

La géométrie de l'espace de rangement 216 permet un enroulement aisé du tuyau 1 autour du réservoir 210 et assure une compacité de l'ensemble du dispositif 2.

L'embase 21 présente, en outre, sur la paroi latérale 215, des moyens de raccordement, sous la forme d'un embout de raccordement 214 du tuyau 1 sur l'embase 21 se situant à un niveau intermédiaire entre la paroi de fond 217, correspondant à un niveau minimum, et le bord supérieur 218 du réservoir 210, correspondant à un niveau maximum. Dans l'exemple illustré, l'embout de raccordement 214 est ici situé environ à distance égale des extrémités supérieure et inférieure de l'embase 21.

La paroi latérale 215 s'étend donc entre la paroi de fond 217 et le bord supérieur 218, sur une hauteur intérieure H. L'embout de raccordement (moyens de raccordement) 214 est placé sensiblement à mi-hauteur, définissant deux hauteurs H1 (entre le niveau inférieur Ninf, correspondant au fond 217, et le niveau intermédiaire Ninter de l'embout) et H2 (entre le niveau intermédiaire Ninter et un niveau supérieur Nsup correspondant au bord supérieur 218, ou à un niveau proche de celui-ci), avec H1 + H2 = H.

Sur ce mode de réalisation, H1 et H2 sont sensiblement égaux. Plus généralement, on peut choisir H1 et H2 de l'ordre de 40 à 60% de H, voire toute autre valeur, en fonction des besoins.

La portion de volume correspondant à la hauteur H2 correspond à la partie assurant le nettoyage (circulation du liquide). La portion de volume correspondant à la hauteur H1 correspond à la partie assurant la stérilisation (circulation de la vapeur).

On note que l'embase peut présenter plusieurs embouts de raccordement de façon à nettoyer plusieurs tuyaux distincts ou bien les tuyaux formant les branches d'un tuyau en Y.

L'embout 214 est configuré pour raccorder une extrémité du tuyau 1 sur l'embase 21 et pour maintenir ce dernier en position lorsque la pression P₁ augmente au sein du réservoir 210 lors du rinçage et de la stérilisation du tuyau 1 (c'est-à-dire lorsque le dispositif 2 est placé dans un four micro-ondes et que le liquide dans le réservoir 210 est porté en ébullition, produisant ainsi de la vapeur). L'extrémité du tuyau 1 est, de préférence, insérée à force sur l'embout 214.

L'embout 214 communique à une extrémité avec le réservoir 210 par le biais d'une ouverture (non visible sur les figures, mais on considère par la suite que cette ouverture est située au même niveau que l'embout 214) ménagée dans la paroi latérale 215, permettant ainsi le passage de l'eau puis de la vapeur depuis le réservoir 210 vers le tuyau 1.

Par ailleurs, l'embase 21 comprend, à son extrémité supérieure, des premiers moyens de solidarisation 213 apte à coopérer avec des seconds moyens de solidarisation 223, situés sur la partie inférieure du bouchon 22, dans le but de fixer le bouchon 22 sur l'embase 21. Ainsi, une fois le bouchon 22 vissé sur l'embase 21, le réservoir 210 est fermé de façon quasi-étanche (l'embout 214 constituant alors le seul passage possible pour l'eau et la vapeur du réservoir 210).

Dans ce mode de réalisation, les moyens de solidarisation réversibles 213, 223 sont formés par un filetage porté par le bouchon 22 apte à coopérer avec un taraudage formé dans l'embase 21 (ou l'inverse).

Ce mode de réalisation est un exemple non limitatif et on comprend que de multiples variantes de solidarisation du bouchon sur l'embase sont possibles (par clipsage ou encliquetage, par exemple).

Dans ce mode de réalisation, le bouchon 22 est également de section ronde et présente un réceptacle (ou enceinte) de collecte 220, destiné à récupérer l'eau souillée provenant du réservoir 210 et ayant circulée dans le tuyau 1 lors des opérations de rinçage et de stérilisation du tuyau 1.

Le bouchon 22 comprend également des moyens de maintien sur lesquels le tuyau 1 peut être fixé de manière amovible.

Ces moyens de maintien se présentent, dans cet exemple, sous la forme d'une encoche, ou ouverture, 222 ménagée dans la partie supérieure de la paroi du réceptacle de collecte 220. Cette ouverture 222 est apte à retenir une extrémité du tuyau 1 de façon à ce que cette extrémité débouche dans le réceptacle de collecte 220.

Le bouchon 22 est, dans cet exemple, fermé par un couvercle 221 amovible cylindrique qui repose sur le haut du bouchon 22 et permet de dissimuler le tuyau 1 lorsque ce dernier est enroulé autour du réceptacle de collecte 220.

Dans ce mode de réalisation, le couvercle 221 présente un évent 225 visant à maintenir une pression P₂ dans le réceptacle de collecte 220 qui soit inférieure à la pression P₁ du réservoir 210 de l'embase 21. Cette différence de pression permet ainsi à l'eau et à la vapeur provenant du réservoir 210 de parcourir le tuyau 1 selon une trajectoire ascendante jusqu'au réceptacle de collecte 220.

On note que la vidange de l'eau souillée contenue dans le réceptacle de collecte 220 (à la fin des opérations de rinçage et de stérilisation, par exemple) nécessite de retirer le couvercle 221.

### 6.2. Mise en place du dispositif de l'invention

On se place par la suite dans le cas d'une première utilisation du dispositif 2 de rinçage et de stérilisation du tuyau 1 de passage du lait d'un tire-lait.

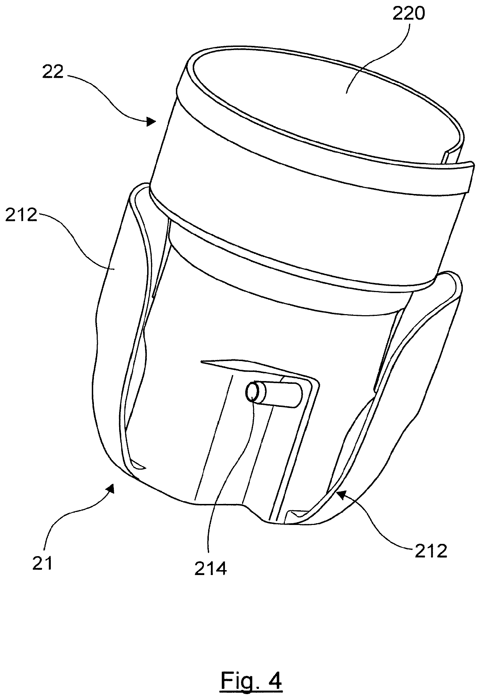

La figure 4 une vue en perspective du dispositif de rinçage et de stérilisation selon l'invention sans le tuyau 1 et sans le couvercle 221.

Dans un premier temps, il est nécessaire de dévisser le bouchon 22 de l'embase 21 pour désolidariser ces deux éléments.

Il est nécessaire ensuite de fixer une première extrémité du tuyau 1 à l'embout 214 (étape E1) avant d'enrouler le tuyau 1 dans l'espace de rangement 216, autour du réservoir 210. Il est à noter que le nombre de tours d'enroulement du tuyau autour du réservoir 210 est fonction de la longueur du tuyau à rincer et stériliser, cet aspect n'ayant aucune influence sur l'efficacité du dispositif 2.

Ensuite, il est nécessaire de remplir d'eau propre le réservoir 210 de l'embase 21 (étape E2).

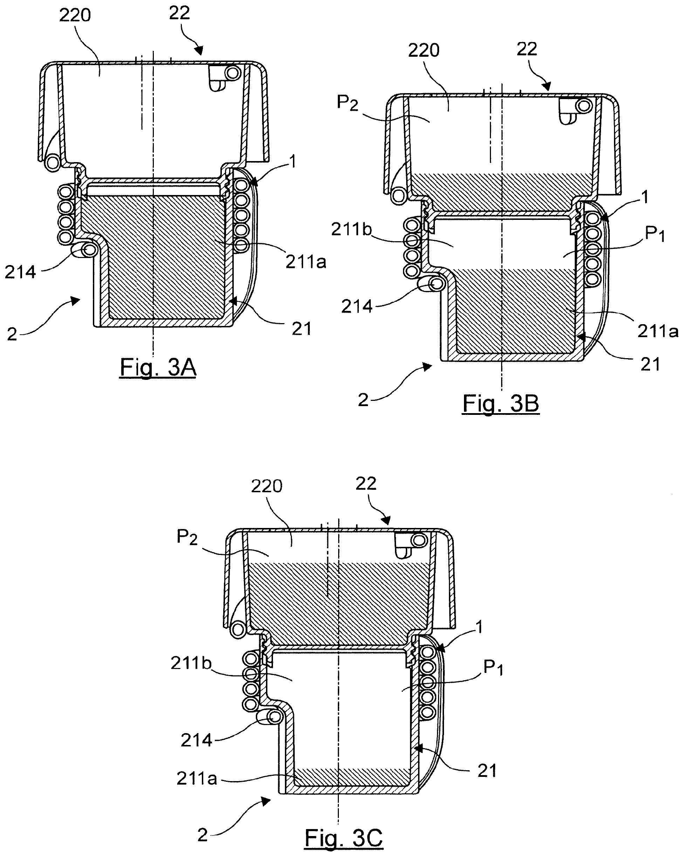

Pour un fonctionnement optimal du dispositif 2, le niveau d'eau est choisi de façon à ce que l'embout de raccordement 214 débouche dans le volume d'eau 211a (figure 3A). L'eau contenue dans le réservoir 210 est utilisée pour procéder au rinçage, puis à la stérilisation du tuyau 1.

Ensuite, on visse le bouchon 22 sur l'embase 21 (étape E3), on enroule le tuyau 1 autour du réceptacle de collecte 220, puis on place la deuxième extrémité du tuyau 1 dans l'ouverture 222 du réceptacle de collecte 220, l'ouverture 222 étant configurée pour maintenir l'extrémité du tuyau 1 en place (étape E4).

On place ensuite le couvercle 221 sur le bouchon 22 de façon à fermer le réceptacle de collecte 220 (le tuyau 1 est alors situé dans l'espace situé entre le réceptacle de collecte 220 et le couvercle 221).

Pour finir, on place le dispositif 2 dans des moyens de chauffage (non représentés) qui sont, préférentiellement, un four à micro-ondes.

Les figures 5A et 5B sont des vues éclatées du dispositif de rinçage et de stérilisation selon l'invention, sans et avec le tuyau 1 respectivement.

### 6.3. Phase 1 : Rinçage du tuyau

Partant de la situation de la figure 3A, le chauffage 61 provoque l'augmentation de la température de l'eau 211a contenue dans le réservoir 210 (qui est étanche) jusqu'à l'ébullition. Le dégagement de vapeur 211b provoque l'augmentation de la pression P₁ à l'intérieur du réservoir 210 (figure 3B).

La pression P₂ dans le réceptacle de collecte 220 du couvercle 221 étant inférieure à la pression P₁ (cette différence de pression est assurée par l'évent 225), la vapeur 211b provoque le déplacement de l'eau 211a dans le tuyau 1 via l'embout 214. L'eau circule dans le tuyau 1 jusqu'à l'extrémité du tuyau 1 placée dans l'ouverture 222 du réceptacle de collecte 220 et se déverse dans le réceptacle de collecte 220.

On comprend aisément que le niveau d'eau 211a dans le réservoir 210 baisse alors progressivement, à mesure que la vapeur 211b pousse l'eau 211a dans le tuyau 1.

Cette approche permet ainsi un rinçage efficace du tuyau 1 à l'eau chaude (étape E5) qui se poursuit jusqu'à ce que le niveau d'eau 211a atteigne le niveau de l'embout 214, comme illustré sur la figure 3B.

En d'autres termes, l'eau chaude 211a élimine les résidus de lait dans le tuyau 1 et est recueillie dans le réceptacle de collecte 220 comme illustré sur la figure 3B.

### 6.4. Phase 2 : Stérilisation du tuyau

Une fois que le niveau d'eau du réservoir 210 est situé en dessous de l'embout de raccordement 214, l'opération de rinçage est terminée et débute alors (étape E6) la stérilisation du tuyau 1 (sous réserve que l'eau 211a soit toujours chauffée).

Durant cette phase de stérilisation, ce n'est plus l'eau du réservoir 210 mais la vapeur d'eau 211b qui circule dans le tuyau 1.

La vapeur collectée dans le réceptacle de collecte 220 est évacuée par le biais de l'évent 225 ou alors retrouve son état liquide et est stockée dans l'enceinte de collecte 220 d'eau souillée (figure 3C).

Cette phase de stérilisation peut être poursuivie jusqu'à ce que le réservoir 210 soit vide ou non.

Ainsi, la vapeur permet une stérilisation efficace du tuyau 1 en éliminant les bactéries ou autres germes résiduels n'ayant pas été éliminés lors de la première étape de rinçage décrite précédemment.

L'étape de stérilisation (E6) peut être suivie d'une et/ou en partie simultanée avec une étape de séchage (E7) du tuyau, par passage de vapeur dans le tuyau.

On note que la vidange (E8) du réceptacle de collecte 220 se fait par simple enlèvement du couvercle 221 et vidage.

### 6.5. Autres aspects et variantes

L'étanchéité de la liaison entre le bouchon 22 et l'embase 21, et donc le réservoir 210, peut être optimisée par l'utilisation d'un ou plusieurs joints d'étanchéité.

Les moyens de solidarisation 213, 223 peuvent être formés par des éléments de clipsage réversible.

Par ailleurs, la paroi latérale 215 peut comprendre une gorge de réception hélicoïdale du tuyau 1 permettant un rangement aisé du tuyau 1.

La position de l'embout de raccordement 214 n'est pas limitée à celle illustrée.

Il peut ainsi être situé plus près de la paroi de fond 217 de l'embase 21 dans le cas où un volume d'eau de rinçage plus important est souhaité, ou bien plus près du bord supérieur 218 de l'embase 21 si la stérilisation est privilégiée.

Par ailleurs, un ou plusieurs évents 225 peuvent être prévus.

Le montage des deux parties 21, 22 du dispositif de l'invention n'est pas limité à celui illustré. Ces deux parties peuvent être montées de façon juxtaposée, et non pas de façon superposée comme illustré sur les figures.

Le réservoir peut être fermé de façon étanche par un bouchon indépendant du réceptacle de collecte.

Le dispositif de l'invention peut ne comprendre qu'un réservoir de liquide auquel est raccordée une extrémité du tuyau à rincer et à stériliser (en d'autres termes, on peut ne pas prévoir de réceptacle de collecte).

Dans ce cas, l'autre extrémité du tuyau peut être placée dans un réceptacle (une assiette par exemple) et l'eau qui parcourt le tuyau peut se déverser dans ce réceptacle, ou être placée directement dans un évier par exemple.

Le dispositif de l'invention peut être utilisé avec tout type de liquide adapté au rinçage et à la stérilisation, comme de l'eau seule (eau minérale ou du robinet), de l'eau mélangée à un nettoyant, ou du nettoyant seul.

Par ailleurs, le réservoir peut présenter une autre forme qu'une forme cylindrique, par exemple une forme sensiblement cubique ou tronconique.

Dans une variante de mise en oeuvre, la connexion ou le branchement du tuyau à nettoyer se fait à l'intérieur du réservoir, le tuyau étant par exemple replié à l'intérieur du réservoir, et reposant par exemple sur une grille suspendue parallèlement à la paroi de fond par exemple. Une ouverture traversante dans le bouchon peut être prévue pour l'autre extrémité, pour évacuer le liquide ayant circulé dans le tuyau.

## Revendications

1. Dispositif (2) de rinçage et de stérilisation d'au moins un tuyau (1) de passage du lait d'un tire-lait, comprenant un réservoir (210) et un bouchon (22) permettant de le fermer,
**caractérisé en ce que** ledit réservoir (210) présente des moyens de raccordement (214) d'une des extrémités du ou d'au moins un desdits tuyaux (1), placés sur une paroi latérale (215) dudit réservoir (210), à un niveau intermédiaire entre la paroi de fond (217) et le bord supérieur (218) dudit réservoir (210),
de façon que, lorsque ledit réservoir (210) contient un liquide comprenant de l'eau et que celui-ci est porté à ébullition, la vapeur produite déplace au moins une partie du volume de liquide dans ledit au moins un tuyau (1) permettant son rinçage, puis, lorsque le niveau dudit liquide a atteint lesdits moyens de raccordement, circule dans ledit au moins un tuyau (1) permettant sa stérilisation.

2. Dispositif (2) selon la revendication 1, **caractérisé en ce que** lesdits moyens de raccordement (214) sont situés à une hauteur comprise entre 40 et 60% de la hauteur totale dudit réservoir (210).

3. Dispositif (2) selon la revendication 1 ou 2, **caractérisé en ce qu'**il comprend en outre un réceptacle de collecte (220) dudit liquide et de la vapeur ayant circulés dans ledit au moins un tuyau (1).

4. Dispositif (2) selon la revendication 3, **caractérisé en ce que** le réceptacle de collecte (220) comprend des moyens de maintien (222) d'une seconde extrémité dudit au moins un tuyau (1) sur ledit réceptacle de collecte (220).

5. Dispositif (2) selon la revendication 4, **caractérisé en ce que** lesdits moyens de maintien (222) se présentent sous la forme d'une ouverture située en partie supérieure dudit réceptacle de collecte (220).

6. Dispositif (2) selon l'une des revendications 3 à 5, **caractérisé en ce que** ledit bouchon (22) porte le ou est solidaire dudit réceptacle de collecte (220).

7. Dispositif (2) selon la revendication 6, **caractérisé en ce que** ledit réservoir (210) comprend des premiers moyens de solidarisation (213) aptes à coopérer avec des seconds moyens de solidarisation (223) portés par ledit bouchon (22).

8. Dispositif (2) selon l'une des revendications 1 à 7, **caractérisé en ce qu'**il comprend une embase (21) portant au moins ledit réservoir (210).

9. Dispositif (2) selon la revendication 8, **caractérisé en ce que** l'embase (21) comprend au moins un élément (212) définissant un espace de rangement (216) dudit au moins un tuyau (1) le long dudit réservoir (210).

10. Dispositif (2) selon l'une des revendications 8 et 9, **caractérisé en ce que** ladite embase (21) présente au moins une gorge apte à maintenir ledit au moins un tuyau (1) enroulé autour dudit réservoir (210).

11. Dispositif (2) selon l'une des revendications 3 à 10, **caractérisé en ce que** le réceptacle de collecte (220) comprend un couvercle (221) amovible.

12. Dispositif (2) selon la revendication 11, **caractérisé en ce que** ledit couvercle (221) comprend au moins un évent (225) débouchant vers l'extérieur dudit dispositif (2).

13. Procédé de rinçage et de stérilisation d'au moins un tuyau (1) de passage de lait d'un tire-lait,
**caractérisé en ce qu'**il comprend une étape de chauffage d'un liquide comprenant de l'eau, contenu dans un réservoir (210) présentant des moyens de raccordement (214) d'une des extrémités du ou d'au moins un desdits tuyaux (1), placés sur une paroi latérale (215) dudit réservoir (210), à un niveau intermédiaire entre la paroi de fond (217) et le bord supérieur (218) dudit réservoir (210),
ladite étape de chauffage portant ledit liquide à ébullition de façon à provoquer la production de vapeur, assurant successivement :
- une étape (E5) de rinçage, par passage d'au moins une partie du liquide dans ledit au moins un tuyau (1) ; puis
- une étape (E6) de stérilisation, par passage de vapeur dans ledit au moins un tuyau (1).

14. Procédé selon la revendication 13, **caractérisé en ce que** ladite étape de stérilisation (E6) est suivie d'une et/ou en partie simultanée avec une étape de séchage (E7) dudit tuyau, par passage de vapeur dans ledit au moins une tuyau (1).

15. Procédé selon l'une quelconque des revendications 13 et 14, **caractérisé en ce qu'**il comprend en outre :
- une étape (E1) de fixation dudit au moins un tuyau (1) sur ledit réservoir (210) par le biais des moyens de raccordements (214) ;
- une étape (E2) de remplissage dudit réservoir (210) avec un volume de liquide (211a) comprenant de l'eau ;
- une étape (E3) d'assemblage dudit réceptacle de collecte (22) avec ledit réservoir (210) ;
- une étape (E4) de solidarisation dudit au moins un tuyau (1) audit réceptacle de collecte (220) par le biais des moyens de maintien (222).

## Patentansprüche

1. Vorrichtung (2) zur Spülung und zur Sterilisation von mindestens einer Leitung (1) für den Milchdurchlauf einer Milchpumpe, umfassend:
einen Behälter (210) und einen diesen zu verschließen ermöglichenden Deckel (22),
**dadurch gekennzeichnet, dass** der Behälter (210) Mittel zum Verbinden (214) eines der Enden der Leitung oder mindestens einer der Leitungen (1) aufweist, die an einer seitlichen Wand (215) des Behälters (210) auf einem mittleren Niveau zwischen der Bodenwand (217) und dem oberen Rand (218) des Behälters (210) angeordnet sind,
derart, dass, wenn der Behälter (210) eine Wasser enthaltende Flüssigkeit beinhaltet und diese zum Sieden gebracht wird, der erzeugte Dampf mindestens einen Teil des Flüssigkeitsvolumens in der mindestens einen Leitung (1) verdrängt, wodurch ihre Spülung ermöglicht wird,
und der erzeugte Dampf dann, wenn das Niveau der Flüssigkeit die Mittel zum Verbinden erreicht hat, in die mindestens eine Leitung (1) strömt, wodurch ihre Sterilisation ermöglicht wird.

2. Vorrichtung (2) nach Anspruch 1, **dadurch gekennzeichnet, dass** die Mittel zum Verbinden (214) auf einer Höhe in einem Bereich von 40 bis 60 % der gesamten Höhe des Behälters (210) angeordnet sind.

3. Vorrichtung (2) nach Anspruch 1 oder 2, **dadurch gekennzeichnet, dass** sie weiterhin ein Auffangbehältnis (220) für die Flüssigkeit und für den Dampf, der durch die zumindest eine Leitung (1) geströmt ist, umfasst.

4. Vorrichtung (2) nach Anspruch 3, **dadurch gekennzeichnet, dass** das Auffangbehältnis (220) Mittel zum Halten (222) eines zweiten Endes der mindestens einen Leitung (1) an dem Auffangbehältnis (220) umfasst.

5. Vorrichtung (2) nach Anspruch 4, **dadurch gekennzeichnet, dass** die Mittel zum Halten (222) sich in Form einer Öffnung, die sich teilweise über dem Auffangbehältnis (220) befindet, darbieten.

6. Vorrichtung (2) nach einem der Ansprüche 3 bis 5, **dadurch gekennzeichnet, dass** der Deckel (22) das Auffangbehältnis (220) trägt oder mit dem Auffangbehältnis (220) verbunden ist.

7. Vorrichtung (2) nach Anspruch 6, **dadurch gekennzeichnet, dass** das Behältnis (210) erste Befestigungsmittel (213) umfasst, die dafür geeignet sind, mit zweiten Befestigungsmitteln (223) zusammenzuwirken, die von dem Deckel (22) getragen werden.

8. Vorrichtung (2) nach einem der Ansprüche 1 bis 7, **dadurch gekennzeichnet, dass** sie ein Unterteil (21) umfasst, das mindestens das Behältnis (210) trägt.

9. Vorrichtung (2) nach Anspruch 8, **dadurch gekennzeichnet, dass** das Unterteil (21) mindestens ein Element (212) umfasst, das einen Stauraum (216) der mindestens einen Leitung (1) entlang des Behältnisses (210) definiert.

10. Vorrichtung (2) nach einem der Ansprüche 8 und 9, **dadurch gekennzeichnet, dass** das Unterteil (21) mindestens eine Hohlkehle aufweist, die dazu geeignet ist, die mindestens eine Leitung (1) um das Behältnis (210) herum gewickelt zu halten.

11. Vorrichtung (2) nach einem der Ansprüche 3 bis 10, **dadurch gekennzeichnet, dass** das Auffangbehältnis (220) einen entfernbaren Verschlussdeckel (221) umfasst.

12. Vorrichtung (2) nach Anspruch 11, **dadurch gekennzeichnet, dass** der Verschlussdeckel (221) mindestens eine Lüftungsöffnung (225) umfasst, die sich nach außerhalb der Vorrichtung (2) öffnet.

13. Verfahren zur Spülung und zur Sterilisation mindestens einer Leitung (1) für den Milchdurchlauf einer Milchpumpe,
**dadurch gekennzeichnet, dass** es einen Schritt des Erwärmens einer Wasser enthaltenden Flüssigkeit umfasst, die in einem Behältnis (210) enthalten ist, welches Mittel zum Verbinden (214) eines der Enden der Leitung oder mindestens einer der Leitungen (1) aufweist, die an einer seitlichen Wand (215) des Behältnisses (210) auf einem mittleren Niveau zwischen der Bodenwand (217) und dem oberen Rand (218) des Behältnisses (210) angeordnet sind,
wobei der Schritt des Erwärmens die Flüssigkeit derart zum Sieden bringt, dass die Erzeugung von Dampf herbeigeführt wird, wodurch sukzessive sichergestellt wird:
- ein Schritt (E5) des Spülens durch Leiten von mindestens einem Teil der Flüssigkeit in die mindestens eine Leitung (1); ferner
- ein Schritt (E6) des Sterilisierens durch Leiten von Dampf in die mindestens eine Leitung (1).

14. Verfahren nach Anspruch 13, **dadurch gekennzeichnet, dass** der Schritt des Sterilisierens (E6) gefolgt wird von einem und/oder teilweise gleichzeitig mit einem Schritt des Trocknens (E7) der Leitung durch das Leiten von Dampf in die mindestens eine Leitung (1).

15. Verfahren nach einem der Ansprüche 13 und 14, **dadurch gekennzeichnet, dass** es weiterhin umfasst:
- einen Schritt (E1) des Festmachens der mindestens einen Leitung (1) an dem Behältnis (210) mittels der Mittel zum Befestigen (214);
- einen Schritt (E2) des Füllens des Behältnisses (210) mit einem Wasser enthaltenden Volumen an Flüssigkeit (211a);
- einen Schritt (E3) des Zusammenfügens des Auffangbehältnisses (222) mit dem Behältnis (210);
- einen Schritt (E4) des Befestigens der mindestens einen Leitung (1) an dem Auffangbehältnis (220) mittels der Mittel zum Halten (222).

## Claims

1. Device (2) for rinsing and sterilising at least one tube (1) enabling the milk of a breast pump to pass therethrough,
comprising a container (210) and a stopper (22) making it possible to close it, **characterised in that** said container (210) has means for connecting (214) one of the ends of the or of at least one of said tubes (1), placed on a side wall (215) of said container (210), at an intermediate level between the bottom wall (217) and the upper edge (218) of said container (210),
in such a way that, when said container (210) contains a water-containing liquid and that latter is brought to boiling, the vapour produced moves at least part of the volume of the liquid into said at least one tube (1) allowing its rinsing, then, when the level of said liquid has reached said means for connecting, flows into said at least one tube (1) allowing its sterilisation.

2. Device (2) according to claim 1, **characterised in that** said means for connecting (214) are located at a height between 40 and 60% of the total height of said container (210).

3. Device (2) according to claim 1 or 2, **characterised in that** it further comprises a receptacle for collecting (220) said liquid and the vapour that flowed into said at least one tube (1).

4. Device (2) according to claim 3, **characterised in that** the receptacle for collecting (220) comprises means for maintaining (222) a second end of said at least one tube (1) on said receptacle for collecting (220) .

5. Device (2) according to claim 4, **characterised in that** said means for maintaining (222) have the form of an opening located in the upper portion of said receptacle for collecting (220).

6. Device (2) according to one of claims 3 to 5, **characterised in that** said stopper (22) carries the or is integral with said receptacle for collecting (220).

7. Device (2) according to claim 6, **characterised in that** said container (210) comprises first means for fastening (213) able to cooperate with second means for fastening (223) carried by said stopper (22).

8. Device (2) according to one of claims 1 to 7, **characterised in that** it comprises a base (21) carrying at least said container (210).

9. Device (2) according to claim 8, **characterised in that** the base (21) comprises at least one element (212) defining a storage space (216) of said at least one tube (1) along said container (210).

10. Device (2) according to one of claims 8 and 9, **characterised in that** said base (21) has at least one groove able to maintain said at least one tube (1) wound around said container (210).

11. Device (2) according to one of claims 3 to 10, **characterised in that** the receptacle for collecting (220) comprises a removable cover (221).

12. Device (2) according to claim 11, **characterised in that** said cover (221) comprises at least one vent (225) that opens outwards of said device (2) .

13. Method for rinsing and sterilising at least one tube (1) enabling the milk of a breast pump to pass therethrough,
**characterised in that** it comprises a step of heating a water-containing liquid, contained in a container (210) that has means for connecting (214) one of the ends of the or of at least one of said tubes (1), placed on a side wall (215) of said container (210), at an intermediate level between the bottom wall (217) and the upper edge (218) of said container (210),
said step of heating bringing said liquid to a boiling point in such a way as to provoke the production of vapour, successively providing:
- a step (E5) of rinsing, by passing at least one portion of the liquid into said at least one tube (1); then
- a step (E6) of sterilising, by passing vapour into said at least one tube (1).

14. Method according to claim 13, **characterised in that** said step of sterilising (E6) is followed by one and/or a simultaneous portion with a step of drying (E7) said tube, by passing vapour in said at least one tube (1) .

15. Method according to any of claims 13 and 14, **characterised in that** it further comprises:
- a step (E1) of fastening said at least one tube (1) onto said container (210) through means for connecting (214);
- a step (E2) of filling said container (210) with a volume of water-containing liquid (211a);
- a step (E3) of assembling said receptacle for collecting (22) with said container (210);
- a step (E4) of fastening said at least one tube (1) to said receptacle for collecting (220) through the means for maintaining (222).
